# DEMANDE DE BREVET EUROPEEN

(11) **EP 0 587 955 A1**
(43) Date de publication de la demande: **23.03.1994**
(21) Numéro de dépôt: 92403156.0
(22) Date de dépôt: 24.11.1992
(51) Int. Cl.: A61F 2/36

(54) **Prothèse de hanche**

(30) Priorité: 15.09.1992 FR 9211345
(71) Demandeur: Choteau, Michel, F-59360 Le Cateau (FR); Debroucker, René, F-62300 Lens (FR); Letendart, Joel, F-59880 Saint Saulve (FR); Mathevon, Henri, F-59140 Dunkerque (FR); Stahl, Philippe, F-59710 Avelin (FR); Vigier, Philippe, F-59830 Louvil (FR)
(72) Inventeur: Choteau, Michel, F-59360 Le Cateau (FR); Debroucker, René, F-62300 Lens (FR); Letendart, Joel, F-59880 Saint Saulve (FR); Mathevon, Henri, F-59140 Dunkerque (FR); Stahl, Philippe, F-59710 Avelin (FR); Vigier, Philippe, F-59830 Louvil (FR)
(74) Mandataire: Lepage, Jean-Pierre

(57) **Abrégé**

Prothèse de hanche (1) pour l'articulation de la tête fémorale de l'os du fémur dans le cotyle de la hanche.

Selon l'invention, elle comprend
- un corps (2) allongé, destiné à venir se loger à l'intérieur du corps et du col du fémur, qui présente à son extrémité supérieure une partie recourbée (5) à l'intérieur de laquelle est ménagé un orifice (7),
- un moyen amovible (11) relié par une de ses extrémités à la partie recourbée (5) et pénétrant dans l'orifice (7)
- une tête fémorale (12) montée librement sur l'autre extrémité du moyen amovible (11) afin de régler l'angle d'ante version.

Application dans le domaine médical et paramédical.

## Description

La présente invention a pour objet une prothèse de hanche pour l'articulation de la tête fémorale de l'os du fémur à l'intérieur du cotyle de la hanche.

Elle trouvera son application dans le domaine médical et paramédical ainsi que dans celui de la fabrication et de la conception de telles prothèses.

Le fémur est un os long qui constitue le squelette de la cuisse en présentant un col et une tête fémorale qui s'articule à l'intérieur d'un cotyle de la hanche. Physiologiquement, ce fémur présente deux constantes anatomiques qui sont :
- un angle d'ante version du col fémoral correspondant à l'inclinaison du plan de la tête du fémur par rapport au plan du fémur ; chez un sujet normal, cet angle est de 10°,
- un angle d'inclinaison du col du fémur sur le fémur ; chez un sujet normal, cet angle est de 130°.

Chez certains sujets, on constate, par exemple avec l'âge, une diminution de la minéralisation du squelette osseux, ce qui le fragilise et peut générer des fractures du col du fémur ou de la tête fémorale.

Chez certains sujets, il se peut également que l'on constate une usure des cartilages de la tête ou du cotyle, ce qui provoque une gêne au niveau de l'articulation.

Il faut alors procéder à la mise en place d'une prothèse de hanche pour remplacer l'articulation de la tête fémorale de l'os du fémur à l'intérieur du cotyle de la hanche.

Pour cela, on coupe la tête fémorale et l'on dispose, à l'intérieur du col et du corps de l'os du fémur, une prothèse constituée par un corps allongé dont l'une des extrémités coopère avec une tête montée libre en articulation dans une cupule fixée sur le bassin.

En pratique, de telles prothèses présentent de nombreux inconvénients et, notamment, celui de n'être pas toujours bien adaptée à la morphologie propre du sujet. La prothèse doit s'adapter au mieux à la cavité fémorale afin de permettre une liberté suffisante à l'articulation de la tête.

Par ailleurs, le corps de la prothèse doit rester immobile à l'intérieur du col et du corps de l'os du fémur et notamment, elle ne doit pas bouger en translation, en rotation ou en inclinaison car toute modification même minime de sa position générerait une luxation.

Pour cela, il est connu de sceller le corps de la prothèse de la hanche à l'intérieur du col et du corps de l'os du fémur par l'intermédiaire d'un ciment polymérisable tel que le polymétacrylate de méthyle qui forme une pâte devenant solide. Or, la réaction de polymérisation du polymétacrylate de méthyle est une réaction exothermique qui brûle momentanément et localement l'os, ce qui risque de provoquer une suppression du tissu fibreux et de ce fait, une raréfaction osseuse. Cette réaction de polymérisation exothermique provoque également une chute de tension artérielle de l'individu, ce qui peut être lui être préjudiciable.

Pour remédier à ces inconvénients, on a pensé à diminuer la quantité de ciment utilisé et notamment, on a pour cela conçu des prothèses qui présentent une forme ovalaire. Toutefois, en pratique, de telles prothèses n'offrent pas un maintien suffisant puisque le contact entre le corps de la prothèse et l'os s'effectue ponctuellement et tangentiellement, ce qui ne permet pas d'assurer un maintien efficace de celle-ci.

Un autre inconvénient des prothèses actuellement utilisées réside dans le fait qu'elles ont une durée de vie en général limitée à dix ou quinze ans, car on a constaté que le ciment polymérisable perdait, avec le temps, de sa cohésion et de sa résistance et se désagrégeait. Il est donc nécessaire de changer totalement la prothèse après cette période, ce qui nécessite une nouvelle intervention sur le patient, intervention qui peut être coûteuse et préjudiciable.

Pour remédier à ces inconvénients, il existe également une possibilité de réaliser des prothèses sur mesure, adaptées à la morphologie propre de chaque patient. Pour cela, par scanner, on mesure et on détermine les dimensions intérieures du fémur et l'on réalise, en fonction de ces données, une prothèse de hanche spécifique à chaque individu.

De telles prothèses sont d'un prix de revient très élevé car elles nécessitent un travail long et complexe pour tout d'abord calculer et dimensionner le fémur et, d'autre part, réaliser une prothèse adaptée à celui-ci.

La présente invention a pour but de remédier aux inconvénients des prothèses actuellement connues en fournissant une prothèse de hanche pour l'articulation de la tête fémorale de l'os du fémur dans le cotyle de la hanche qui peut s'adapter à toutes les maladies et les situations anatomiques en prévoyant une possibilité de réglage de l'angle d'ante version.

Un autre but de la prothèse de hanche conforme à l'invention réside dans le fait qu'elle est parfaitement maintenue en position à l'intérieur du col et du corps de l'os du fémur sans nécessiter l'utilisation d'une quantité importante de ciment, voire sans utiliser de ciment.

Un autre but de la prothèse conforme à l'invention réside dans le fait qu'elle permet d'obtenir un contact planéiforme avec l'os plutôt qu'un contact ponctuel, ce qui accroît son maintien.

Un avantage de la prothèse de hanche conforme à l'invention réside dans le fait que l'on prévoit des revêtements de traitement spécifiques de sa surface afin d'améliorer son accrochage et sa fixation à l'os.

Un autre avantage de la prothèse de hanche conforme à l'invention réside dans le fait que la partie inférieure de son corps est prévue pour s'éloigner de l'os en cas de déformation, ce qui évite le blocage distal.

Un autre avantage de la prothèse de hanche conforme à l'invention réside dans le fait que l'on peut régler l'angle d'ante version selon différentes positions d'une manière simple et aisée, ce qui facilite la mise en place de la prothèse quelle que soit la conformation du patient.

Un autre avantage de la prothèse conforme à l'invention réside dans le fait qu'en cas d'usure, l'on ne doit remplacer uniquement qu'une partie de celle-ci correspondant aux zones d'articulation qui ont une fatigue. Cette deuxième intervention sera donc beaucoup plus facile et beaucoup moins onéreuse que la deuxième intervention dans l'art antérieur, dans laquelle il fallait changer totalement et complètement la prothèse.

Un autre but de la prothèse de hanche conforme à l'invention réside dans le fait qu'elle peut être réalisée en série pour un faible cout de fabrication.

D'autres buts et avantages de la présente invention apparaîtront au cours de la description qui va suivre, qui n'est donnée qu'à titre indicatif et qui n'a pas pour but de la limiter.

A cette fin, la prothèse de hanche pour l'articulation de la tête fémorale de l'os du fémur dans le cotyle de la hanche est caractérisée par le fait qu'elle comprend :
- un corps allongé, destiné à venir se loger à l'intérieur du corps et du col du fémur, qui présente à son extrémité supérieure une partie recourbée à l'intérieur de laquelle est ménagé un orifice,
- un moyen amovible relié par une de ses extrémités à la partie recourbée et pénétrant dans l'orifice,
- une tête fémorale montée librement sur l'autre extrémité du moyen amovible afin de régler l'angle d'ante version.

L'invention sera bien comprise à la lecture de la description suivante, accompagnée des dessins en annexe parmi lesquels :
- la figure 1 est une vue schématique qui illustre, en position éclatée, les différents éléments constituant la prothèse de hanche conforme à l'invention,
- la figure 2 est une vue schématique qui illustre une prothèse de hanche conforme à l'invention en position montée d'utilisation,
- la figure 3 est une vue schématique qui montre une variante de réalisation de la pièce de liaison qui présente l'une de ses extrémités recourbées,
- la figure 4 est une vue schématique qui illustre une position de la pièce de liaison selon une orientation correspondant à une fermeture d'angle qualifiée de varus,
- la figure 5 est une vue schématique qui illustre la pièce de liaison disposée selon une orientation correspondant à un accroissement de l'angle selon une position qualifiée de valgus,
- les figures 6 et 7 sont des vues schématiques qui illustrent deux positions de réglage de la pièce de liaison selon une disposition en retro version,
- les figures 8 et 9 sont des vues schématiques qui illustrent une position de la pièce de liaison selon deux positions de réglage correspondant à l'ante version.

La présente invention a pour objet une prothèse de hanche pour l'articulation de la tête fémorale de l'os du fémur dans le cotyle de la hanche, qui trouvera son application notamment dans le domaine médical, paramédical, ainsi que dans celui de la conception et de la fabrication de ces prothèses.

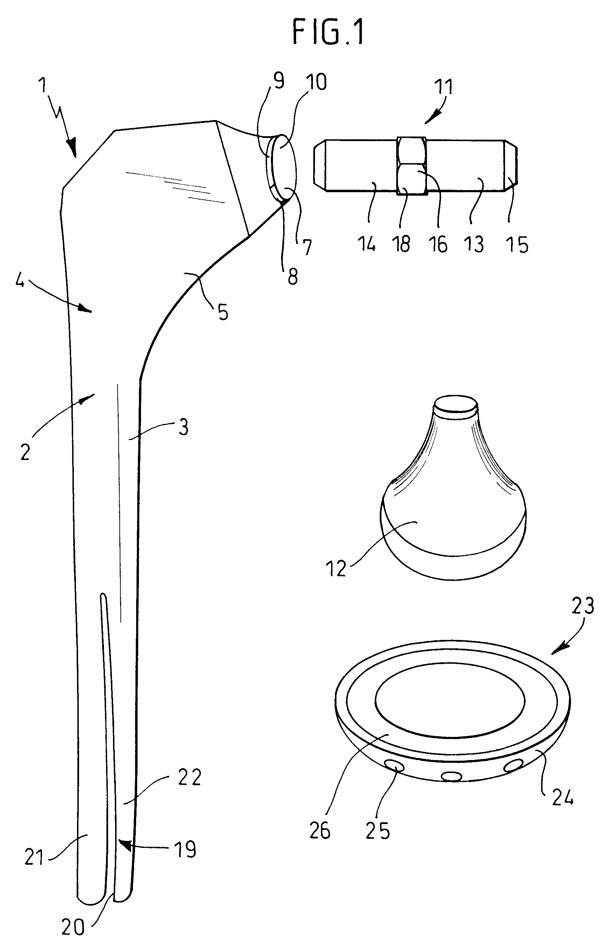

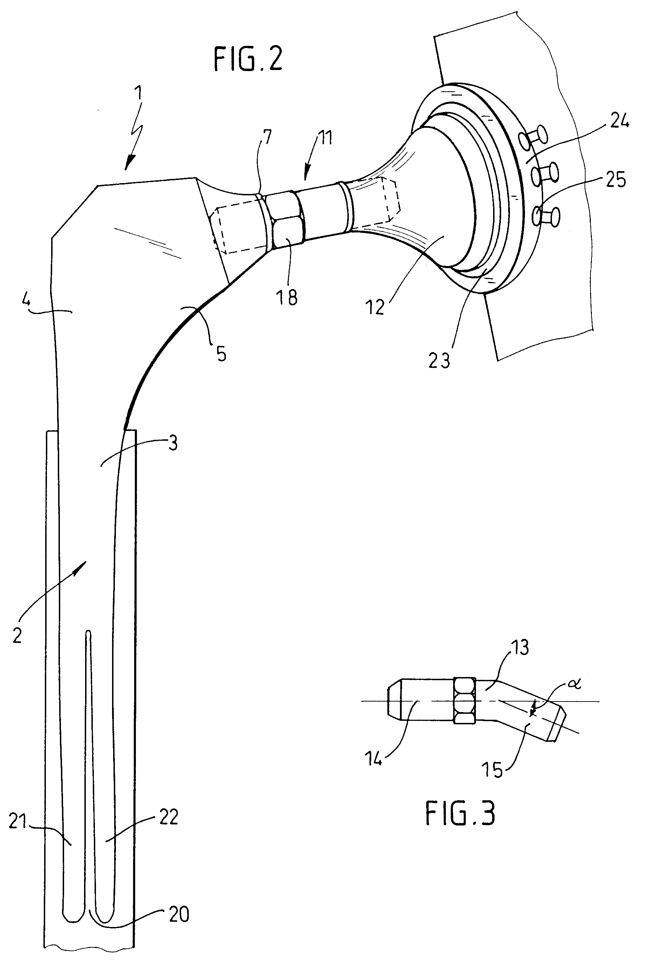

En se référant aux figures 1 et 2, on voit une prothèse de hanche (1) conforme à l'invention, qui présente un corps allongé (2) dont la section n'est pas elliptique. On a au contraire prévu un contour de facettes (4) constituant les trois côtés d'un trapèze (4a, 4b, 4c) qui se prolongent ensuite par un tronçon d'ellipse (4d).

Dans un mode d'exécution préféré de la variante décrite, on a prévu que les facettes (4a, 4b, 4c) de la partie recourbée (5) étaient prolongées sur une partie de la longueur du corps allongé (2).

Ainsi, grâce à cette forme particulière du corps (2), en position montée, cette prothèse présente plusieurs zones de contact avec l'os, selon des plans, ce qui facilite son maintien dans la position désirée en évitant notamment les phénomènes de déplacements en rotation ou en translation de la prothèse (1) susceptibles de générer des luxations.

Le corps (2) présente à son extrémité supérieure une partie recourbée (5) selon un angle sensiblement égal à l'angle d'inclinaison du corps de l'os du fémur.

L'orifice (7) est ménagé sensiblement dans la partie médiane de la partie recourbée (5) selon un angle par rapport à l'horizontale correspondant sensiblement à l'angle d'ante version. La périphérie (8) de la paroi interne (9) présente une répartition régulière de pans (10). Dans un mode de réalisation privilégié, on a prévu un nombre de pans égal à six, ce qui donnera une possibilité de six réglages de l'angle d'ante version mais on pourrait naturellement les réduire ou les augmenter.

La prothèse de hanche (1) comporte des moyens (11) de liaison et de réglage de l'angle d'ante version d'une tête fémorale (12) constituée par une pièce (13) dont chaque extrémité (14, 15) présente une section tronconique.

Cette pièce de liaison (13) peut présenter dans une forme de réalisation une configuration dans laquelle les deux extrémités (14, 15) sont en regard l'une de l'autre par rapport à un plan médian vertical. Dans une variante de réalisation telle qu'illustrée à la figure 3, une autre configuration de cette pièce (13) consiste à réaliser l'extrémité (15) inclinée par rapport à l'axe médian horizontal.

La pièce de liaison (13) présente également une partie centrale (16) qui comporte des pans (18) répartis sur sa périphérie selon une conformation et une dimension permettant son introduction à l'intérieur de l'orifice (7). La position de la pièce de liaison (13) par rapport à la partie recourbée (5) est déterminée par la rotation de la pièce (13) avant son introduction dans l'orifice (7). L'introduction de l'extrémité (14) permet de guider la pièce (13) mais ne l'immobilise pas en rotation et c'est seulement en poussant les pans (18) dans les pans (10) que l'on obtient cette immobilisation en rotation.

Les pans (18) permettent d'assurer un réglage de l'angle d'ante version et de l'angle d'inclinaison de la prothèse de hanche (1) afin de s'adapter en fonction des besoins à la morphologie du patient et du diagnostic du praticien.

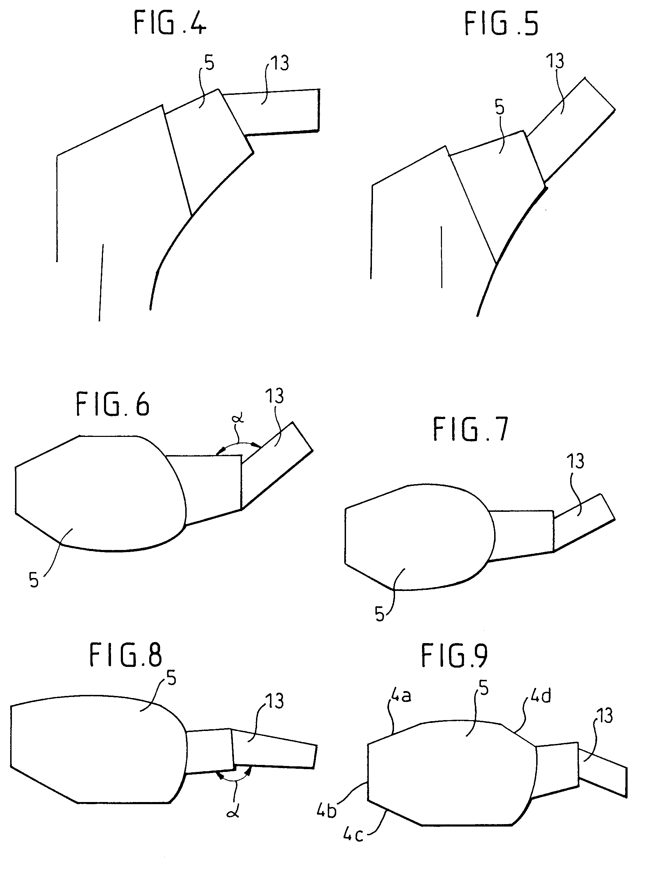

Si l'on utilise une pièce de liaison (13) telle qu'illustrée à la figure 3, c'est-a-dire présentant un angle α différent de zéro, on peut obtenir une augmentation de cet angle en orientant la pièce de liaison (13) dans une position telle qu'illustrée à la figure 5. On obtiendra une diminution de l'angle en positionnant la pièce de liaison telle qu'illustrée à la figure 4. Le praticien peut ainsi obtenir en fonction de ses besoins un accroissement ou une diminution de l'angle d'ante version selon une plage angulaire variant de 5 à 15° et de préférence selon un angle de 8° afin d'éviter les risques de luxation.

Le praticien pourra, s'il le souhaite, placer dans la prothèse et selon une variante de l'invention, une pièce de liaison (13) présentant un angle α égal à zéro, c'est-à-dire avec des extrémités (14 et 15) dans le prolongement l'une de l'autre. Cette pièce sera conformée de la même manière et présentera notamment des pans (18) similaires, susceptibles de s'adapter dans l'orifice (7).

En fonction du nombre de pans (18) et (10), il est possible d'obtenir un nombre plus ou moins important de possibilité d'orientation de la pièce de liaison (13). C'est ainsi que dans la forme de réalisation représentée, on a choisi deux possibilités de réglage de l'angle d'ante version, telles qu'illustrées aux figures 6 et 7. Les autres positions de réglage de l'orientation de la pièce de liaison (13) correspondent à deux positions de l'angle de retro version, telles qu'illustrées aux figures 8 et 9.

Comme on l'a déjà exposé, la présente prothèse pourra être réalisée en série, c'est-à-dire sans examen particulier au scanner du patient. Cette fabrication en série permettra de couvrir toutes les plages d'utilisation de la prothèse qui devra naturellement pouvoir s'adapter à toutes les tailles des patients et à toutes les morphologies. L'invention permet de mettre en oeuvre aisément ces caractéristiques puisque l'on pourra dimensionner à souhait la prothèse, tant au niveau de la partie recourbée (5) que de la pièce amovible (13) ou bicône.

A titre d'exemple, on a réalisé les variantes suivantes : sept tailles fémorales, quatre tailles de bicône :
- l'un normal, c'est-à-dire avec les extrémités alignées et l'angle α égal à zéro,
- l'autre avec un angle α égal à 8°.

Chacun de ces bicônes est également prévu avec des longueurs différentes des extrémités (14 et 15)
L'extrémité inférieure (19) du corps allongé (2) comporte une fente verticale (20) s'étendant sur une partie de sa longueur afin d'éviter, en cas de déplacement de la prothèse, un contact de celle-ci avec l'os générant un blocage distal. En effet, sous l'action d'une translation provoquée par un enfoncement de la prothèse, les deux parties (21 et 22) ménagées de part et d'autre de la fente (20) s'éloignent de l'os.

Il est en effet important, dans la partie basse de la prothèse, au niveau du corps allongé (2), de ne pas réaliser une fixation mécanique sur l'os ; on a constaté que dans la partie diaphysaire dans le cylindre de l'os, la prothèse doit être libre et non solidarisée à l'os pour éviter une déminéralisation à la partie supérieure et provoquer la dévitalisation, ce qui risquerait d'engendrer des déplacements intempestifs.

Au contraire, dans la partie métaphysaire, c'est-à-dire dans la partie recourbée (5), la prothèse doit être bloquée et c'est dans cette zone que l'on cherchera à la faire se solidariser à l'os pour obtenir un bon positionnement. A titre indicatif, la partie métaphysaire représente en hauteur un tiers de l'ensemble de la prothèse alors que la partie diaphysaire représente les deux tiers.

La prothèse de hanche (1) comporte également une cupule (23) destinée à servir de logement à la tête (12) réalisée de préférence en un matériau résistant tel qu'en céramique ou en alliage métallique du type chrome cobalt.

La cupule externe (24) confectionnée en un matériau métallique résistant présente au voisinage de sa calotte une répartition régulière d'orifices (25) destinés à recevoir des vis pour assurer sa fixation sur l'os de la hanche. A l'intérieur de la cupule externe (24) est placée une cupule interne (26) réalisée en un matériau qui présente un état de surface lisse pour obtenir un couple de frottement faible avec la tête (12). A titre d'exemple, il peut s'agir d'un insert réalisé en polyéthylène ou en tout autre matériau de synthèse présentant des propriétés équivalentes.

Ainsi, selon l'invention, il est possible d'obtenir une articulation de la tête (12) à l'intérieur de la cupule interne (26) qui reproduit fidélement le mouvement naturel de l'os sur le bassin.

Un avantage de la présente invention réside dans le fait que, si l'on observe une usure de la prothèse de hanche, il ne sera plus nécessaire de remplacer l'ensemble de la prothèse (1) mais seulement la cupule interne (26) et éventuellement la tête (12), ce qui limite le coût et la durée de l'intervention tout en diminuant les risques pour le patient.

Ainsi, de par sa forme, cette prothèse de hanche (1) est bien adaptée à la cavité fémorale et elle ne peut se déplacer en translation ou en rotation.

Il faut également noter que, pour améliorer 1'accrochage de la prothèse de hanche (1) à l'intérieur du col et du corps de l'os du fémur, on peut prévoir différents moyens et notamment des revêtements de surface.

Tout d'abord, on projette dans la partie supérieure du corps (2), c'est-à-dire dans la partie métaphysaire, de l'hydroxyapatite qui est une molécule de calcium se fixant sur l'acide oxaloglutamique présent dans l'os pour provoquer une soudure biologique. Cette projection d'hydroxyapatite doit être effectuée uniquement dans la partie métaphysaire du corps (2) afin d'éviter que le corps allongé (2) ne soit maintenu dans le bas car il y aurait alors un risque de déminéralisation de l'os. C'est la raison pour laquelle, de préférence, on traite uniquement le tiers supérieur du corps (2) de la prothèse de hanche (1).

On pourra aussi éventuellement recouvrir cette partie métaphysaire du corps (2) par un sablage au corindon afin d'obtenir une irrégularité de la surface de la prothèse, facilitant son accrochage.

Ce traitement aura pour but de provoquer des déformations, telles que des irisations sur la surface de la partie recourbée (5) et par exemple dans les parties (4a, 4b, 4c) ou sur le tronçon d'ellipse (4d). Ce traitement permettra ainsi un meilleur positionnement de la prothèse et une meilleure fixation à l'os dans la partie métaphysaire.

Le corps (2) de la prothèse est, quant à lui, réalisé dans un matériau tel qu'en titane ou en un alliage métallique par exemple chrome, cobalt ou bien encore en céramique ou en tout autre matériau présentant des caractéristiques adaptées à la confection de prothèses.

Ainsi, selon l'invention, il est possible d'obtenir une mise en place et un maintien de la prothèse de hanche (1) sans nécessiter l'utilisation de ciment ou en en mettant en oeuvre une quantité faible

Bien entendu, d'autres formes de réalisation de la présente invention auraient pu être envisagées sans pour autant sortir du cadre de celle-ci.

## Revendications

1. Prothèse de hanche (1) pour l'articulation de la tête fémorale de l'os du fémur dans le cotyle de la hanche, caractérisée en ce qu'elle comprend :
- un corps (2) allongé, destiné à venir se loger à l'intérieur du corps et du col du fémur, qui présente à son extrémité supérieure une partie recourbée (5) à l'intérieur de laquelle est ménagé un orifice (7),
- un moyen amovible (11) relié par une de ses extrémités à la partie recourbée (5) et pénétrant dans l'orifice (7),
- une tête fémorale (12) montée librement sur l'autre extrémité du moyen amovible (11) afin de régler l'angle d'ante version.

2. Prothèse de hanche, selon la revendication 1, caractérisée en ce que les moyens (11) de liaison et de réglage de la tête (12) sont constitués par une pièce (13) dont chaque extrémité (14, 15) présente une section tronconique.

3. Prothèse de hanche, selon la revendication 2, caractérisée en ce que l'une des extrémités (15) tronconique est inclinée d'un angle α par rapport à l'axe médian horizontal.

4. Prothèse de hanche, selon la revendication 2, caractérisée en ce que la pièce (13) présente une partie centrale (16) qui comporte des pans (18) répartis sur sa périphérie afin de permettre le réglage de l'angle d'ante version.

5. Prothèse de hanche, selon la revendication 1, caractérisée en ce que l'orifice (7) de la partie recourbée (5) présente sur au moins une partie de sa périphérie (8) des pans (10) de forme et de répartition complémentaires de ceux de la pièce de liaison (13) afin de permettre son ajustement et son orientation.

6. Prothèse de hanche, selon la revendication 1, caractérisée en ce que l'extrémité inférieure (19) du corps (2) présente une fente (20) verticale s'étendant sur une partie de sa longueur.

7. Prothèse de hanche, selon la revendication 1, caractérisée en ce que la partie recourbée (5) est recouverte d'un matériau dur et résistant.

8. Prothèse de hanche, selon la revendication 1, caractérisée en ce que la partie recourbée (5) est recouverte d'un matériau facilitant la soudure avec l'os.

9. Prothèse de hanche, selon la revendication 1, caractérisée en ce que le corps allongé (2) présente des multifacettes destinées à venir se loger à l'intérieur du col et du corps du fémur afin de prévenir des mouvements en rotation.
